# EUROPEAN PATENT APPLICATION

(11) **EP 4 296 834 A1**
(43) Date of publication of application: **27.12.2023**
(21) Application number: 22180764.7
(22) Date of filing: 23.06.2022
(51) Int. Cl.: G06F 3/04815, G06Q 10/10, G06T 19/00, G16H 50/50, G16H 80/00, H04N 7/15

(54) **RENDERING A VIRTUAL MODEL**

(71) Applicant: Koninklijke Philips N.V., 5656 AG Eindhoven (NL)
(72) Inventor: BUIL, Vincentius Paulus, Eindhoven (NL); GEURTS, Lucas Jacobus Franciscus, Eindhoven (NL); HABETS, Hugo, 5656AG Eindhoven (NL); DE HAAN, Marla, 5656AG Eindhoven (NL); VAN LEENGOED, Marleen Johanna Jacoba, 5656 AG Eindhoven (NL); LAUTE, Niels, 5656 AG Eindhoven (NL); SYED, Aly Aamer, 5656 AG Eindhoven (NL)
(74) Representative: Philips Intellectual Property & Standards

(57) **Abstract**

Proposed concepts thus aim to provide schemes, solutions, concepts, designs, methods and systems pertaining to generating virtual model renderings in extended reality for viewing by participants of a meeting. In particular, embodiments of the invention propose the ability to switch between two rendering modes, based on receiving either a grouped visualization request or a personal visualization request. Responsive to receiving a grouped visualization request the virtual model is rendered in a central location for viewing by all participants. Responsive to receiving the personal visualization request, the virtual model is rendered for each participant at respective participant locations. In this way, a virtual model to be viewed at a collaborative meeting may be more appropriately rendered based on viewing needs of the participants.

## Description

### FIELD OF THE INVENTION

The present invention relates to the field of virtual model rendering in extended reality, and in particular to the field of virtual model renderings for viewing by participants of a meeting.

### BACKGROUND OF THE INVENTION

Immersive, interactive augmented reality (AR), mixed reality (MR) and virtual reality (VR) assisted visual collaborative meetings enable people to communicate and collaborate with one another in innovative ways. This provides a useful mode for sharing complex ideas, which may not be achieved by traditional methods. Extended reality (i.e. AR, MR and VR) assisted collaborative meetings have the potential to be used in a variety of different domains, such as schools, workplaces, broadcasting, and tourism. Furthermore, such meetings have the benefit that they may be held at a variety of different, remote locations, while immersing participants in a way that may create a sense of the participants being together, even when they are physically distant.

Typical extended reality meetings allow remote participants to work together on a centralized virtual model, with each participant seeing other participants as avatar in the same extended reality environment. In certain meetings (i.e. a virtual classroom session, or a multi-disciplinary team meeting) the participants may want to explore different elements of the virtual model (i.e. other viewpoints, other data layers, and other locations outside the current focus of the virtual model). However, existing virtual meetings are not well suited to facilitate such explorations, as a central synchronized virtual is not ideal for detailed personal inspection of the virtual models. Indeed, this problem is exacerbated for meetings of larger groups.

### SUMMARY OF THE INVENTION

The invention is defined by the claims.

According to examples in accordance with an aspect of the invention, there is provided a method for generating virtual model renderings in extended reality for viewing by participants of a meeting, the method comprising:
obtaining a mode instruction comprising at least one of a grouped visualization request and a personal visualization request;
responsive to obtaining the mode instruction comprising a grouped visualization request, rendering a virtual model in a central location corresponding to an area viewable by each of the participants, such that each participant sees a same version of the virtual model; and
responsive to obtaining the mode instruction comprising the personal visualization request, for each of the participants, rendering the virtual model in a respective participant location corresponding to an area in front of the participant, such that each participant initially sees a same version of the virtual model.

Proposed concepts thus aim to provide schemes, solutions, concepts, designs, methods and systems pertaining to generating virtual model renderings in extended reality for viewing by participants of a meeting. In particular, embodiments of the invention propose the ability to switch between two rendering modes, based on receiving either a grouped visualization request or a personal visualization request. Responsive to receiving a grouped visualization request the virtual model is rendered in a central location for viewing by all participants. Responsive to receiving the personal visualization request, the virtual model is rendered for each participant at respective participant locations. In this way, a virtual model to be viewed at a collaborative meeting may be more appropriately rendered based on viewing needs of the participants.

During a meeting, it is beneficial for a virtual model to be rendered in a central location. In this way, all participants of a meeting may view the virtual model in a same location, while each participant may also be aware of the other participants. This allows collaborative working between multiple participants of the meeting, and is particularly ideal when the virtual model may not be the sole focus of attention for the participants.

Embodiments of the invention are based on the realization, in certain circumstances, the rendering of the virtual model in a central location may not be ideal. For example, when close inspection of the virtual model is required, having the model rendered in a central location may be difficult, especially for a meeting with many participants (e.g. in a classroom/lecture theatre related environment). Thus, the invention provides a means to switch between one mode in which the virtual model is rendered centrally, to another mode where the virtual model is rendered for each participant in a dispersed manner.

More specifically, when a personal visualization request is received, the virtual model is rendered for each participant. As such, the virtual reality model is rendered in an area corresponding to an area in front of the participant. This may be more convenient for close personal inspection by the individual participants.

In other words, the invention provides a method for rendering virtual model in such a way that the virtual reality model is positioned appropriately. In some circumstances it is appropriate to render the virtual model centrally, whereas in other situations a personal/dispersed rendering may be more appropriate.

Indeed, the invention leverages the advantages that conducting meetings in an extended reality environment may provide (i.e. remote meetings, ability to control surroundings, etc.), while being conscious that the area/place in the extended reality environment that a virtual model is rendered should be dynamic. The dynamic rendering may be based on the needs of the participant and a presenter, and the context of the meeting. In any case, the invention provides the flexibility to switch between these two scenarios, by the receiving of a mode instruction specifying at least one of a grouped visualization request and a personal visualization request.

In some embodiments, the method may further comprise obtaining an exploration request for at least one of the participants. Further, responsive to obtaining the exploration request, for each of the at least one participant associated with the exploration request, the virtual model may be rendered in the respective participant location such that the participant sees a personalized version of the virtual model.

Accordingly, individual participants may focus of aspects of the virtual model that are of interest to them. For example, if the virtual model is representative of an anatomy of a person, then some participants may be interested in the cardiovascular system, wile others may be interested in the muscular system. In other cases, it may be preferable that participants want to view the back of the model, while some wish to view the front of the virtual model.

As such, the rendering of the virtual model may be appropriate for individual viewing when in the participant location.

In this case, the method may further comprise a step of obtaining a manipulation instruction associated with the at least one participant, the manipulation instruction describing a modification to the virtual model. The personalized version of the virtual model for the at least one participant may be based on the manipulation instruction. The manipulation instruction may comprise at least one of a scale change, an orientation change, and a viewpoint change.

Thus, when enabled, the participant may manipulate/change/reorient the virtual model as they see fit.

In some cases, the personalized version of the virtual model for the at least one participant may be further based on a modification permission parameter associated with the at least one participant, the modification permission parameter describing a limitation of the modification of the virtual model by the at least one participant.

It may prove particularly beneficial to define the bounds by which the participant can change the virtual model for viewing. For example, if the meeting is in a classroom environment a presenter/teacher may wish to restrict participants to only view certain parts of the model. In other cases, this may be used to ensure privacy or protection of sensitive information. In yet another example, different participants may have access to different aspects of the virtual reality model, such as when parts are unsuitable for viewing by children.

As such, the modification permission parameter may be based on a context of the meeting. In this way, the modification parameter may be automatically set, reducing the need for user/presenter intervention.

Further, the modification permission parameter may be further based on a system map describing associations between different aspects of the virtual model.

In an embodiment, the mode instruction may be obtained from a presenter, the presenter being a participant responsible for the meeting.

In this way, the presenter may determine when is best to switch between a centralized view and dispersed/personal view of the virtual model.

In some embodiments, the same version of the virtual model may be based on a presenter manipulation instruction obtained from the presenter, the presenter manipulation instruction describing a modification to the virtual model.

As such, the presenter(s) may be solely responsible for manipulating/changing the virtual model when in the central location, and may also control the virtual model when in the participant locations, so as to show the participants what they wish.

The mode instruction may comprise at least one of the grouped visualization request, the personal visualization request and a mixed visualization request. In this case, the method may further comprise a step, responsive to obtaining the mode instruction comprising the mixed visualization request, for each of the participants, of rendering the virtual model in the respective participant location such that each participant initially sees a same version of the virtual model, and rendering the virtual model in the central location such that each participant also sees the same version of the virtual reality model.

By the addition of the possibility of a mixed visualization request, the virtual model may be rendered both in a central location viewable by all participants, and also at their individual participant locations. In this way, the benefits of both visualization modes may be provided. This may be particularly advantageous, for example, when each participant virtual model is being manipulated by the individual participants, while the presenter displays an overview on the centrally-based virtual model.

In some embodiments, the method may further comprise, responsive to obtaining the mode instruction comprising the personal visualization request, for at least one participant, rendering the virtual model in the participant locations corresponding to each of the participants, such that the at least one participant sees a view of the virtual model rendered for each of the participants in the respective participant locations.

In this way, a participant(s) (i.e. a presenter, all participants) may see the view that each other participant is seeing. This may aid in collaborative working by improving ease of access to the version of the virtual model that is rendered for each participant. Furthermore, it may greatly aid the supervision and teaching purposes, by presenting the views of each participant to the potential teacher/supervisor.

The virtual model may represent an anatomical object. In this case, the modification permission parameter may be based on an anatomical system map describing associations between different aspects of the anatomical object.

On particularly beneficial use case is in the medical domain, where experts from a variety of fields may be observing a 3D representation of an anatomy in the form of the virtual model.

According to further aspects of the invention, there is provided a computer program comprising computer program code means adapted, when said computer program is run on a computer, to implement a method for generating virtual model renderings in extended reality for viewing by participants of a meeting.

According to yet another aspect of the invention, there is provided a system for generating virtual model renderings in extended reality for viewing by participants of a meeting, the system comprising:
an interface configured to obtain a mode instruction comprising at least one of a grouped visualization request and a personal visualization request;
a central control element configured, responsive to obtaining the mode instruction comprising a grouped visualization request via the interface, to render a virtual model in a central location corresponding to an area viewable by each of the participants, such that each participant sees a same version of the virtual model; and
a dispersed control element configured, responsive to obtaining the mode instruction comprising the personal visualization request via the interface, for each of the participants, to render the virtual model in a respective participant location corresponding to an area in front of the participant, such that each participant initially sees a same version of the virtual reality model.

In some embodiments, the interface may be further configured to obtain an exploration request for at least one of the participants. The system may further comprise a modification unit configured, responsive to obtaining the exploration request via the interface, for each of the at least one participant associated with the exploration request, to render the virtual model in the respective participant location such that the participant sees a personalized version of the virtual model.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figs. 1A-1C depict renderings of a virtual model at a number of different locations, responsive to obtaining a mode instruction;
Figs. 2A-2D present system instruction flow diagrams for a number of scenarios related to embodiments of the invention;
Fig. 3 presents an anatomic system map alongside a simplified representation of the anatomy of a heart;
Fig. 4 is a simplified overview of systematized nomenclature of medicine-clinical terms;
Fig. 5 presents a flow diagram of a method for generating virtual model renderings in extended reality for viewing by participants of a meeting according to an embodiment;
Fig. 6 presents a simplified block diagram of a system for generating virtual model renderings in extended reality for viewing by participants of a meeting according to an embodiment; and
Fig. 7 is a simplified block diagram of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

It should be understood that the Figures are merely schematic and are not drawn to scale. It should also be understood that the same reference numerals are used throughout the Figures to indicate the same or similar parts.

The invention proposes concepts for generating virtual model renderings in extended reality for viewing by participants of a meeting according to an embodiment. More specifically, rendering is performed in response to a mode instruction including a grouped visualization request and/or a personal visualization request. In this way, the rendering is performed in a central location viewable by all participants of the meeting, and/or in individual participant locations. Thus, the invention provides a tool that may provide appropriate rendering of the virtual model depending upon a context of the meeting, or presenter/participant preferences.

Proposed concepts are based on the realisation that there exists a present need for a more effective method for rendering virtual reality models for collaborative working (i.e. a meeting) in an extended reality environment. Specifically, there are certain circumstances where a virtual model should be rendered in a central location (i.e. for direct presentation) and where the virtual model should be rendered in a participant location (i.e. for detailed personal inspection). Thus, proposed concepts facilitate the switching between at least two rendering modes.

Moreover, certain embodiments allow the personal modification of the virtual reality models for individual inspection by participants. This allows individuals to explore/investigate the virtual model independently, while the invention still allows for overall control of the virtual model by a person responsible for the meeting (i.e. a presenter).

Yet further embodiments ensure that all personal virtual models rendered in participant locations are viewable by other participants. In this way, it may be easier to discuss points of interest within the virtual reality model being viewed by other participants, as well as have a general awareness of other participant's inspections. This may be particularly useful for collaborative working, or when supervision is required.

By way of explanation, applications of extended reality, including augmented reality (AR), virtual reality (VR) and mixed reality (MR), are known in a variety of fields. For example, in the medical domain extended reality is already used to support training, diagnosis, treatment planning, live surgery and patient communication. Indeed, extended reality proves particularly beneficial in multi-disciplinary team meetings and medical training in a classroom setting.

However, in these cases it is difficult to render a collaborative experience where participants may communicate face to face, while each have the same viewpoint/same version of the virtual model. To overcome this issue, a personal copy of the virtual model at a central stage/central location may be rendered, such that each participant experiences a synchronized orientation (i.e. a same viewpoint of the virtual model, without occupying the same space). This solution may be utilised according to embodiments of the current invention.

Furthermore, a presenter may wish to discuss the virtual model (i.e. a 3D or 4D model) with participants of the meeting. It may be the case that specific points of interest of the virtual model are highlighted by the presenter during discussion. However, it may also be the case that individual participants may wish to explore/investigate different elements of the virtual model. This may include viewing the virtual model from other viewpoints, observing other data layers of the model, and perhaps even other locations outside the current zoom level of the virtual model.

By way of example, when a medical observes a virtual model representing the anatomy of a subject, a (partial) anatomy may be observed. However, some experts may wish to observe the cardiovascular aspects of the anatomy, while others may wish to observe the muscular parts of the anatomy. By way of further example, some experts may wish to observe a blood flow function, while other experts may wish to observe the electrophysiological function of an anatomy.

Such investigations by different participants may also be beneficial for the presenter of the meeting, in order to get a full perspective discussion to the virtual model they are presenting.

Typical solutions to collaborative meetings involving virtual models are not well suited to facilitate such explorations. Indeed, a central, synchronized visualization of a virtual model is suboptimal for detailed personal inspection of the volume, in particular for larger groups. This is because it is impractical/uncomfortable for a large group of people to gather around one virtual mode. While it may be possible to provide each participant with a single centrally placed personal version of the virtual model for individual exploration, this may lead to a lack of awareness between participants, regarding their individual exploration of different areas.

Accordingly, embodiments of the invention provide a solution to the above mentioned problem by initially providing each participant with a rendering of the virtual model, in which initially the viewpoint, orientation and scale is (continuously) synchronized to that of the meeting master. In other words, a same version of the virtual model is presented to each of the participants.

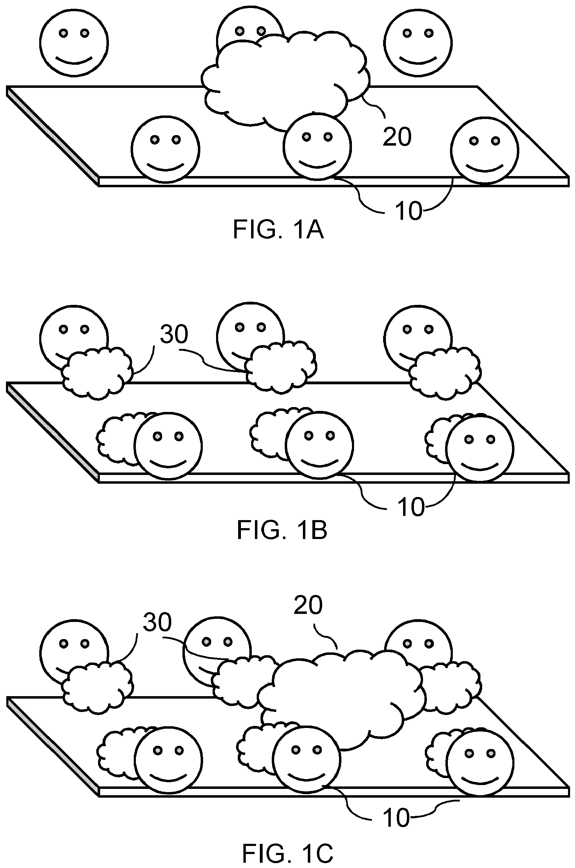

It is then possible to switch (i.e. with the permission of the presenter/meeting master) between a grouped visualization mode (where all virtual models are centrally co-located), a personal visualization mode (where each copy is conveniently in front of the participant), and a mixed mode including both. These modes are presented visually in Figs. 1A-1C.

Fig. 1A depicts participants 10 of a meeting observing the virtual model 20 in a central location corresponding to an area viewable by each of the participants 10. In this case, each participant 10 sees a same version of the virtual model 20.

Generally, this means that each participant 10 may see the virtual model from a same viewpoint, orientation and scale. The viewpoint, orientation and scale may be controlled by a presenter. As an alternative, the central visualization of the virtual model may be rendered in such a way that each participant 10 views the central object from their own viewpoint. In other words, each participant may view the virtual model 20 rendered in a central location at a different viewpoint, orientation and scale, much like a group of participants 10 viewing a real-world model would experience.

Fig. 1B depicts participants 10 of a meeting observing the virtual model 30 in a participant location corresponding to an area in front of each respective participant 10 (i.e. in a location individually viewable by the participant 10). In this case, the participant 10 may see the same version of the virtual reality model 30. In alternative modes/embodiments, the participant 10 may see a personalized version of the virtual reality model 30. Put another way, either the version of the virtual model 30 is synchronized with all other participants 10, or the virtual model 30 is individually investigable by the participant 10 (i.e. with the permission of the presenter). In yet further embodiments, each participant 10 may be able to see each other participant's 10 corresponding virtual reality model 30 in their respective participant locations.

Fig. 1C depicts participants 10 of a meeting observing the virtual reality model 20, 30 in both a central location, as well as in their individual participant locations. In this case, the version of the virtual model 20, 30 (i.e. the viewpoint, zoom level etc.) may be different in the different locations, or may be the same.

In some embodiments, it may be possible to switch between a synchronized and exploratory viewing modes (i.e. based on a permission/request from a presenter). The view may then be re-synchronized with the presenter/another participant when individual investigation/exploration is concluded.

In this case, the manipulation/modification to the virtual model view by the participant during exploratory viewing may be limited to contextually relevant content of the meeting. For example, if during a medical team meeting, exploration may be restricted to clinically relevant content in the virtual model. Clinical relevance may be determined via anatomic, pathologic, and clinical pathway knowledge graphs related to the meeting topic, and may be further constrained by a participant's expertise domain and/or access rights to certain information.

The invention may be embodied, in some cases by the following components. Firstly, an extended reality storage element may be provided to contain information needed for rendering the virtual model, as well as data that identifies access permissions/modification permission parameters.

A control element (which may be part of the extended reality system) may control how the virtual models are displayed to the participant, and what actions/modifications/manipulations on these models are permitted. This may be achieved by an embedded algorithm and data available to it from the storage element.

Thus, when a participant (i.e. the presenter) issues a command to the system to disperse into individual virtual models for the participants (i.e. an exploration request), a personal version of the virtual model in a participant location may be rendered. The control system may receive commands to manipulate these individual virtual models (i.e. a manipulation instructions) from the participants, and may alter the personal version of the virtual model accordingly.

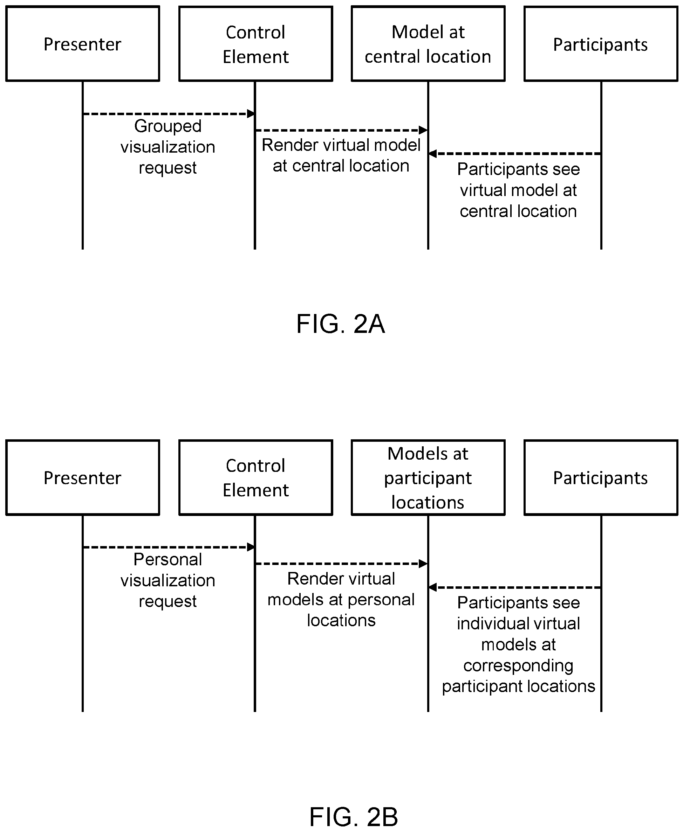

Moving onto Figs. 2A-2D, there is presented four scenarios involving a presenter and participants of the meeting. This includes the rendering of a central virtual model observable by each participant; the rendering of dispersed, personal/individual versions of the virtual model for each participant; the manipulation of the personal versions of the virtual model based on commands and modification permission parameters; and the return to the same version for each individual participant when the presenter initiates the request.

In the following, these scenarios are described in detail.

As depicted in Fig. 2A, in scenarios conducive with some embodiments of the invention, the presenter initiates the presentation of a virtual model in a central location, such that it is positioned centrally amongst all participants, by communicating a grouped visualization request. Each participant sees a same version of the virtual model (i.e. the virtual model as viewed from a same viewpoint). In other words, each participant can see the virtual model from the same perspective, in one central location.

In this scenario, the central virtual model is presented from content available from an extended reality storage element. Further, only the presenter may interact with (i.e. manipulate, modify and change) the central virtual model. The actions performed are copied to the individual copies of the participant, to enable that they all keep seeing the same version of the virtual model from the same perspective.

As depicted in Fig. 2B, in some embodiments of the invention, the presenter may initiate the transition to individual/personal, dispersed copies of the virtual model at participant locations, by communicating a personal visualization request. Optionally, the central virtual model, where each participant sees the same version of the virtual model (i.e. synchronized with the presenter) may remain present as a second rendering for all participants. The individual version of the virtual model may be dependent on data of the extended reality storage element. Still, only the presenter/meeting leader may interact with the centrally rendered virtual model. Their actions are copied to the individual copies of the participant, to enable that they all keep seeing the same volume from the same perspective.

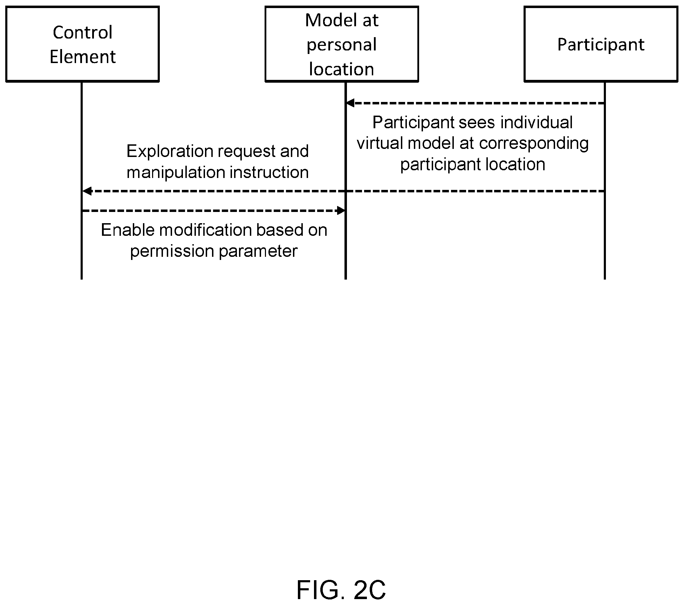

Turning to Fig. 2C, in some scenarios, the presenter may disable the synchronization of the virtual models rendered in the participant locations by communicating an exploration request. Accordingly, the participants may explore their own personal volumes and execute their own manipulations. In this way, the participants may see different versions of the virtual reality model.

Thus, a participant may manipulate/change/modify the rendered virtual model. For example, the participant may zoom in/out, rotate, etc. Further, a participant may inspect other information about the virtual model that they are seeing. In some embodiments, a control element may mediate the actions that the participant is executing on the virtual model, using modification permission parameters associated with the participant. In this way, there are restrictions imposed on the actions performed by the participant. In this way, the participant may only access relevant information

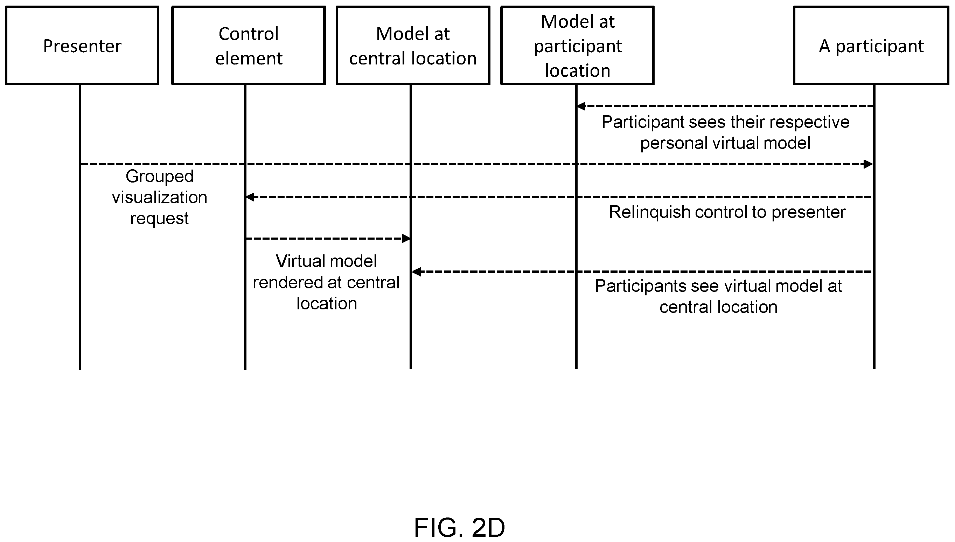

In Fig. 2D, there is depicted a scenario where the participant relinquishes individual control of the virtual model, essentially undoing the exploration request. In this scenario, the participant is initially working with/viewing their respective version of the virtual model at their corresponding participant location. The presenter (or in some cases the participant) may request grouped visualization. Thus, a same version of the virtual model is once again presented in a central location for each participant.

Furthermore, in some embodiments, when a participant views the virtual model in a participant location, they may add annotations/comments to the virtual model. Thus, when the scenario depicted in Fig. 2D occurs, such annotations may be served, and/or merged/visualized alongside the virtual model rendered in the central location. This may depend on various view setting determined automatically, or set by a presenter/other participant.

It may be noted that, in some embodiments, a participant may temporarily take the role of presenter. This may be useful if a participant wishes to show a specific finding, or even share their own data layer as well. In this case the participant's interactions with their own personal version of the virtual model may temporarily be synchronized to the copies of the other participants and of the presenter. The presenter may take back control again whenever needed.

The modification of the personal version of the virtual reality model (when in an exploration mode responsive to an exploration request) may be dependent on modification permission parameters. Such parameters may be specific to the participant, and may also be dependent upon the context of the meeting.

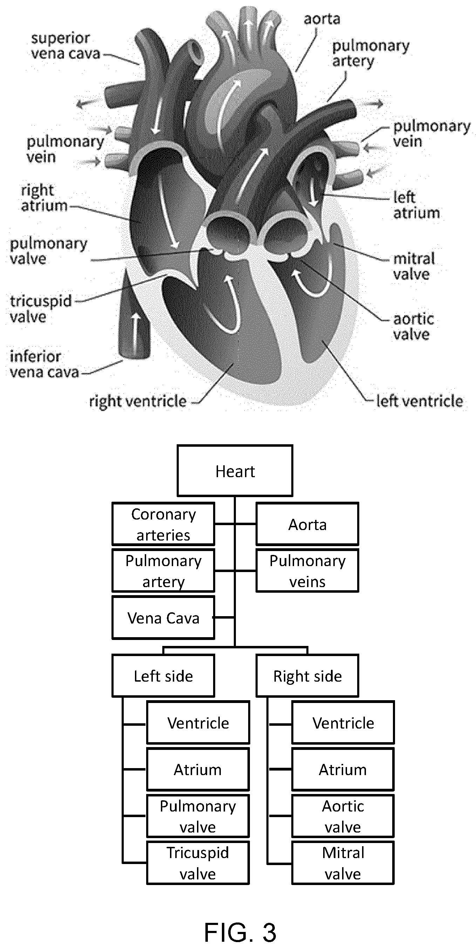

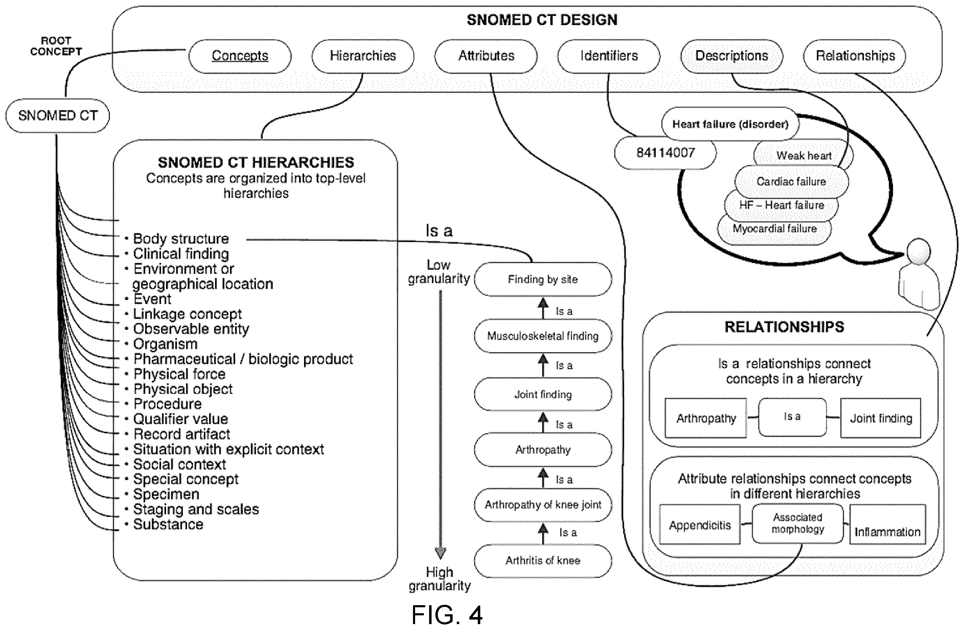

Figs. 3 and 4 relate to concepts relevant to the determination of whether a manipulation of the virtual model by a participant is allowed in the context of a medical meeting (i.e. when the virtual model is of an anatomy). Specifically, Fig. 3 presents a simplified, labelled representation of a heart, as well as an anatomical system map describing links between aspects of the depicted heart.

In this case, clinical relevance (and therefore the relevance of a potential exploration/manipulation instruction) may be determined via an anatomic, pathologic, and clinical pathway knowledge graph(s) related to the meeting topic. Participant manipulation instructions to be executed may be further constrained by a participant's expertise domain and/or access rights to certain information.

For example, when determining whether a manipulation instruction should be executed, an anatomical system map (such as in Fig. 3) may be used in combination with pathological knowledge to determine which parts of the virtual model may be inspected by the participants. Indeed, by way of a specific example, when reviewing a calcified aorta, a pathological knowledge base could determine that it is not necessary to allow exploration beyond the heart as a whole. This may mean that: (i) observation/rendering of the vasculature further away from the heart may only be allowed after further permission; (ii) observation/rendering of the lungs, breasts, ribs, spine, or other nearby organs may also be prohibited for privacy, ethical or legal reasons (i.e. possibly dependent on clearances given by the subject or representative); and (iii) observation/rendering of prosthetics/implants, unless relevant for the current investigation, may also not be enabled.

Furthermore, permission parameters may also make use of a participants disciplinary background, or access rights based on his/her ID and/or a disciplinary nomenclature map that can tell who has access to a certain piece of information. This to allow access to different layers of information and details, such as other volumetric scans, views, personal annotations, lab test data, sample data, etc. Certain data may contain jargon that can lead to confusion or misinterpretation by other disciplines. Sharing such data with other participants in the meeting is therefore only possible by the specific discipline, accompanied with clarifying explanations.

An anatomical system map, such shown in Fig. 3, may be constructed from a medical ontology such as for example the Systematized Nomenclature of Medicine-Clinical Terms (SNOMED CT) as depicted in Fig. 4. Alternatively, the anatomical system map may be constructed from the Ontology for General Medical Science (OGMS). SNOMED CT provides the core general terminology for electronic health records, and includes clinical findings, symptoms, diagnoses, procedures, body structures, organisms, substances, pharmaceuticals, devices and specimens.

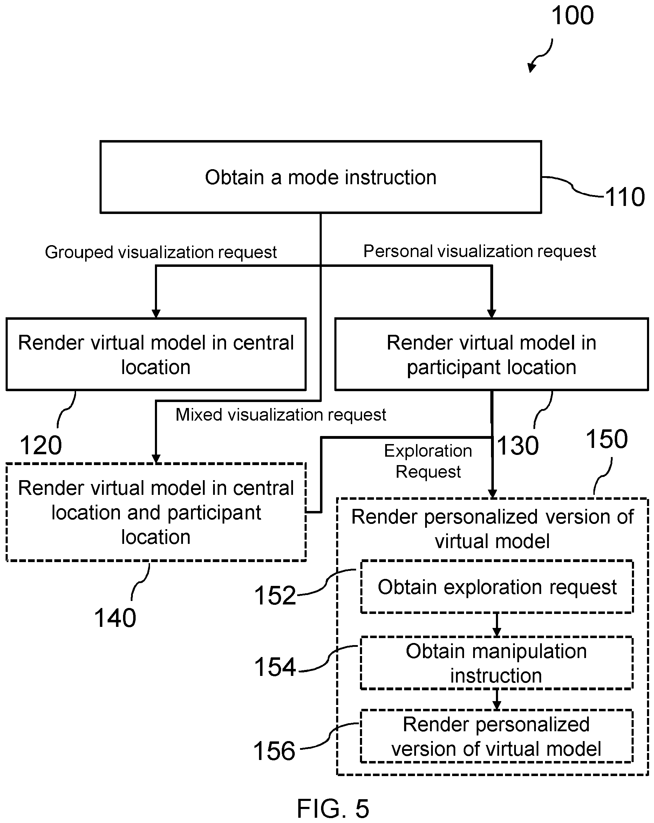

Moving onto Fig. 5, there is presented a flow diagram of a method 100 for generating virtual model renderings in extended reality for viewing by participants of a meeting according to an exemplary embodiment. In other words, the depicted method 100 describes how a virtual model may be rendered in an extended reality environment, in a way which is suitable for viewing by participants (e.g. observers, presenters etc.). The meeting may have many participants, and as such the method may be suitable for rendering a virtual reality model for a classroom, collaborative meeting, or any situation where it may be beneficial to discuss a model with multiple individuals.

At step 110, a mode instruction is obtained. The mode instruction comprises at least one of a grouped visualization request/instruction and a personal visualization request/instruction. The mode instruction may be obtained from a designated administrator (i.e. a presenter or compere of the meeting), or may be obtained from other participants of the meeting. Alternatively, the mode instruction may be automatically generated based on a context of the meeting, thus ensuring an appropriate visualization request is received.

At step 120, responsive to the received mode instruction comprising the grouped visualization request, a virtual model is rendered in a central location. The central location corresponds to an area viewable by each of the participants, such that each participant sees a same version of the virtual model.

Put another way, when a grouped visualization is received, the rendering may be performed in a grouped mode. This means that the virtual model is rendered in a central location, which corresponds to a portion that may be observed by all active participants. For example, this may be the front of a lecture theatre, or the centre of a conference table.

In this mode, the virtual model is rendered for each participant, such that each participant initially sees a same version of the virtual model. This means that each participant may see the virtual model from the same perspective/viewpoint, independent on their physical position and/or position in the extended reality environment. In this way, each participant may appreciate what a presenter/other participant is observing, leading to a more effective collaborative meeting environment.

At step 130, responsive to obtaining the mode instruction comprising the personal visualization request, the virtual model is rendered for each participant in a respective participant location.

The participant location corresponds to an area in front of the respective participant. That is the participant location is a location/position for rendering appropriate for an individual participant to personally inspect the virtual model. This may not necessarily mean that the virtual model is rendered directly in front of the participant, but instead in a location suitable for observance by the participant.

Responsive to the personal visualization request, the virtual model is rendered such that each participant initially sees a same version of the virtual model. Put another way, each participant sees the virtual model from the same viewpoint, orientation, zoom level etc. This may be synchronized with another participant, or a presenter/controller of the meeting.

In this way, personal/close inspection of the virtual model may be more comfortably be achieved by all participants at the same time than when the virtual model is rendered in a central location.

To reiterate, in some embodiments the same version of the virtual model is synchronized with a virtual model associated with a presenter of the meeting. Indeed, in this case the rendered virtual model for each participant may be based on a presenter manipulation instruction obtained from the presenter, the presenter manipulation instruction describing a modification to the virtual model. In this way, the presenter may alter the model, and the version of the virtual model rendered for each participant will be the same as the presenter's. This may enable more effective presenting and collaborative working.

In this case, the presenter manipulation instruction may comprise at least one of a scale change, an orientation change, and a viewpoint change. Indeed, the manipulation instruction may comprise any instruction that alters the virtual model.

Optionally, the mode instruction may also comprise a mixed visualization request. Thus, at step 140, responsive to obtaining the mode instruction comprising the mixed visualization request, the virtual model may be rendered in a central location and at each participant location.

More specifically, the same version of the virtual model is rendered, for each of the participants, in the respective participant location such that each participant initially sees a same version of the virtual model, and in the central location such that each participant also sees the same version of the virtual reality model.

Thus, each participant may see at least a central virtual model leading to associated presenting benefits, while also seeing a personal virtual model leading to associated personal inspection benefits.

In this case, the central virtual model may be synchronized to the version of the virtual model controlled by a presenter/an individual participant. The personal virtual model may be manipulated, such that each participant views a render of their personal version of the virtual model in the participant location. This manipulation will be described in more detail below. However, for now it should be appreciated that the render of the virtual model in the central location and the participant location may be the same, or may differ in certain circumstances.

Moreover, and in order to realise some collaborative working benefits, each participant may also see the virtual model rendered in each other participant location corresponding to other participants. Thus, it may be straightforward to note what point of interest in the model other participants are looking at, or gesturing to.

Put another way, responsive to obtaining the mode instruction comprising the personal visualization request, for at least one participant the virtual model is rendered in the participant locations corresponding to each of the participants, such that the at least one participant sees a view of the virtual model rendered for each of the participants in the respective participant locations.

Indeed, this may only be enabled for a presenter of the meeting, who requires supervisory capacity. In this way, individual participant privacy may be ensured.

In some embodiments, optional step 150 may be included. This involves rendering a personalized version of the virtual model in the participant location for each corresponding participant. As such, more detailed personal inspection of the virtual model may be enabled.

More specifically, at step 152, an exploration request for (or indeed, from) at least one of the participants is obtained. This may be received from a presenter consenting to the personal exploration of the virtual model for at least one of the participants, or may be a request from a participant (that may be approved/disapproved by the presenter, or automatically based on a meeting context, or participant details).

Thus, responsive to obtaining the exploration request, for each of the at least one participant associated with the exploration request, the virtual model is rendered in the respective participant location such that the participant sees a personalized version of the virtual model. This is shown in step 156.

The personalized version may be a version dictated by an external source (i.e. a presenter), or it may bee based on preferences/instructions of the participant to which it is associated.

In the latter case, a manipulation instruction associated with the at least one participant (in an exploration mode) is obtained, as shown in step 154. The manipulation instruction describes a modification/change/manipulation of the virtual model. Indeed, in an embodiments the manipulation instruction may comprise at least one of a scale change, an orientation change, and a viewpoint change.

Accordingly, the personalized version of the virtual model for the at least one participant is based on the manipulation instruction. For example, if the manipulation instruction specifies a rotation of 90 degrees in a vertical axis, the rendered personalized version of the virtual model will be rotated 90 degrees compared to an initial version of the virtual model.

Moreover, as discussed above, the extent to which a participant may manipulate the virtual model may be restricted.

Thus, in some embodiments, the personalized version of the virtual model for the at least one participant is further based on a modification permission parameter (i.e. a rule governing allowed manipulations) associated with the at least one participant. The modification permission parameter describes a limitation of the modification of the virtual model by the at least one participant.

By way of example, it may be beneficial to specify that some participants are not permitted to access certain aspects of the virtual model for security and/or privacy reasons. Further, the modification permission parameter may enable control of the exploration of the virtual model by a presenter. This may be advantageous in a classroom setting, where control over the actions of certain participants may be needed.

The modification permission may apply to each participant individually, or may be a blanket rule for all of the participants. In some embodiments, the modification permission parameter is based on a context of the meeting. In this way restrictions may be automatically applied, such that participants may be restricted to viewing aspects of the virtual model that are relevant to the meeting,

Furthermore, the modification permission parameter may be further based on a system map describing associations between different aspects of the virtual model. Thus, it may be quantified how different aspects of the model relate and are relevant to the context of the meeting.

By way of specific example, in some embodiments the virtual model may represent an anatomical object, and the meeting may be a meeting between various medical-related personnel. When this is the case, the modification permission parameter may be based on an anatomical system map describing associations between different aspects of the anatomical object. Thus, relevance to the meeting topic (i.e. a subject's heart issues) of the virtual model may be ensured (i.e. by restricting virtual model manipulations such that the heart remains the focus of attention).

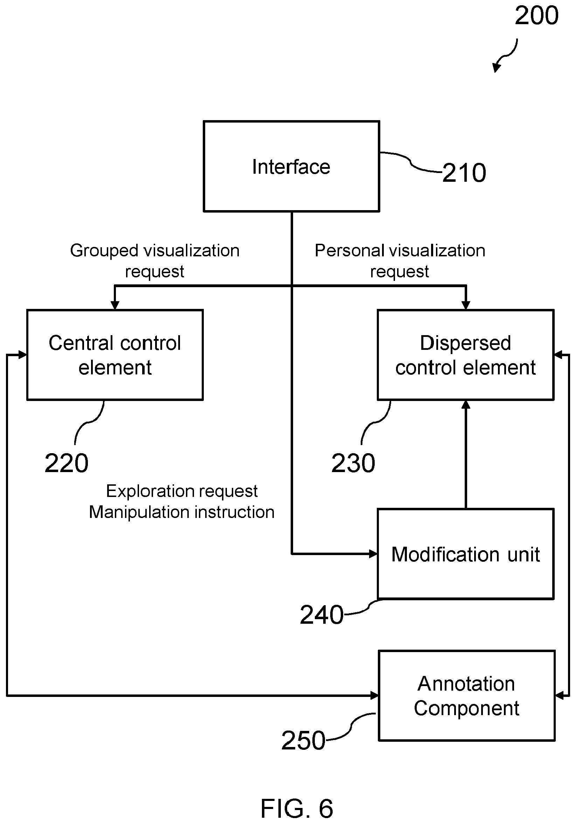

Fig. 6 depicts a simplified block diagram of a system 200 for generating virtual model renderings in extended reality for viewing by participants of a meeting. The system 200 comprises an interface 210, a central control element 220 and a dispersed control element 230. Optionally, the system may further comprise a modification unit 240 and/or an annotation component 250.

The interface 210 is configured to obtain a mode instruction comprising at least one of a grouped visualization request and a personal visualization request. Further, the interface 210 may be configured to obtain an exploration request manipulation instructions and modification permission parameters.

The central control element 220 is configured, responsive to obtaining the mode instruction comprising a grouped visualization request via the interface 210, to render a virtual model in a central location corresponding to an area viewable by each of the participants, such that each participant sees a same version of the virtual model.

The dispersed control element 230 is configured, responsive to obtaining the mode instruction comprising the personal visualization request via the interface 210, for each of the participants, to render the virtual model in a respective participant location corresponding to an area in front of the participant, such that each participant initially sees a same version of the virtual reality model.

It should be noted that the central control element 220 and dispersed control element 230 may be implemented, for example, within an extended reality actuator (i.e. a headset, glasses, etc.), or may be implemented remotely. In any case, the dispersed control element 230 and central control element 220 control the rendering of the virtual model, such that the extended reality actuator presents the virtual model to the participant(s).

The (optional) modification unit 240 is configured, responsive to obtaining the exploration request via the interface 210, for each of the at least one participant associated with the exploration request, to render the virtual model in the respective participant location such that the participant sees a personalized version of the virtual model. In some embodiments, the modification unit 240 may then present the personalized version to the dispersed control element for rendering.

Finally, the annotation component 250 is configured to edit/annotate/comment the virtual models at participant locations responsive to instructions of the corresponding participant. Then, responsive to a grouped visualization request, the annotations of the multiple participants may be merged and stored centrally in a memory unit (not shown). The stored annotations may then be used to annotate/comment/edit the virtual model rendered in a central location by the central control element 220, and/or be used to annotate/comment/edit the virtual model rendered by the dispersed control element 230.

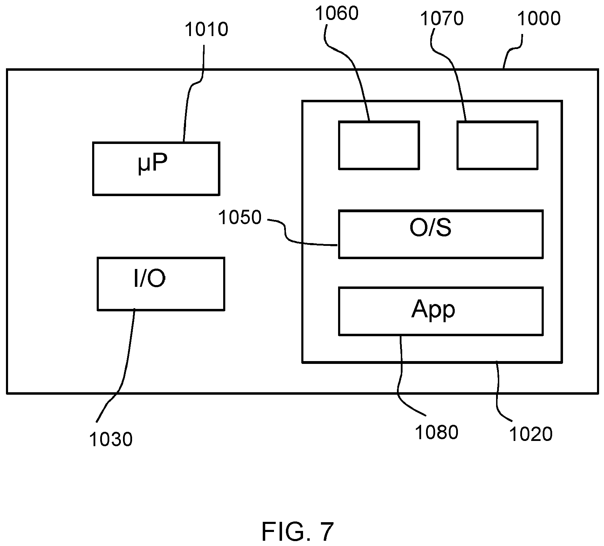

Fig. 7 illustrates an example of a computer 1000 within which one or more parts of an embodiment may be employed. Various operations discussed above may utilize the capabilities of the computer 1000. For example, one or more parts of a system for generating virtual model renderings in extended reality for viewing by participants of a meeting may be incorporated in any element, module, application, and/or component discussed herein. In this regard, it is to be understood that system functional blocks can run on a single computer or may be distributed over several computers and locations (e.g. connected via internet).

The computer 1000 includes, but is not limited to, PCs, workstations, laptops, PDAs, palm devices, servers, storages, and the like. Generally, in terms of hardware architecture, the computer 1000 may include one or more processors 1010, memory 1020 and one or more I/O devices 1030 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 1010 is a hardware device for executing software that can be stored in the memory 1020. The processor 1010 can be virtually any custom made or commercially available processor, a central processing unit (CPU), a digital signal processor (DSP), or an auxiliary processor among several processors associated with the computer 1000, and the processor 1010 may be a semiconductor based microprocessor (in the form of a microchip) or a microprocessor.

The memory 1020 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 1020 may incorporate electronic, magnetic, optical, and/or other types of storage media. Note that the memory 1020 can have a distributed architecture, where various components are situated remote from one another, but can be accessed by the processor 1010.

The software in the memory 1020 may include one or more separate programs, each of which comprises an ordered listing of executable instructions for implementing logical functions. The software in the memory 1020 includes a suitable operating system (O/S) 1050, compiler 1060, source code 1070, and one or more applications 1080 in accordance with exemplary embodiments. As illustrated, the application 1080 comprises numerous functional components for implementing the features and operations of the exemplary embodiments. The application 1080 of the computer 1000 may represent various applications, computational units, logic, functional units, processes, operations, virtual entities, and/or modules in accordance with exemplary embodiments, but the application 1080 is not meant to be a limitation.

The operating system 1050 controls the execution of other computer programs, and provides scheduling, input-output control, file and data management, memory management, and communication control and related services. It is contemplated by the inventors that the application 1080 for implementing exemplary embodiments may be applicable on all commercially available operating systems.

Application 1080 may be a source program, executable program (object code), script, or any other entity comprising a set of instructions to be performed. When a source program, then the program is usually translated via a compiler (such as the compiler 1060), assembler, interpreter, or the like, which may or may not be included within the memory 1020, so as to operate properly in connection with the O/S 1050. Furthermore, the application 1080 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 1030 may include input devices such as, for example but not limited to, a mouse, keyboard, scanner, microphone, camera, etc. Furthermore, the I/O devices 1030 may also include output devices, for example but not limited to a printer, display, etc. Finally, the I/O devices 1030 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 1030 also include components for communicating over various networks, such as the Internet or intranet.

If the computer 1000 is a PC, workstation, intelligent device or the like, the software in the memory 1020 may further include a basic input output system (BIOS) (omitted for simplicity). The BIOS is a set of essential software routines that initialize and test hardware at start-up, start the O/S 1050, and support the transfer of data among the hardware devices. The BIOS is stored in some type of read-only-memory, such as ROM, PROM, EPROM, EEPROM or the like, so that the BIOS can be executed when the computer 800 is activated.

When the computer 1000 is in operation, the processor 1010 is configured to execute software stored within the memory 1020, to communicate data to and from the memory 1020, and to generally control operations of the computer 1000 pursuant to the software. The application 1080 and the O/S 1050 are read, in whole or in part, by the processor 1010, perhaps buffered within the processor 1010, and then executed.

When the application 1080 is implemented in software it should be noted that the application 1080 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 1080 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods described in relation to Fig. 5, and the systems described in relation to Fig. 6, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagram Fig. 6 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A method (100) for generating virtual model renderings in extended reality for viewing by participants of a meeting, the method comprising:
obtaining (110) a mode instruction comprising at least one of a grouped visualization request and a personal visualization request;
responsive to obtaining the mode instruction comprising a grouped visualization request, rendering (120) a virtual model in a central location corresponding to an area viewable by each of the participants, such that each participant sees a same version of the virtual model; and
responsive to obtaining the mode instruction comprising the personal visualization request, for each of the participants, rendering (130) the virtual model in a respective participant location corresponding to an area in front of the participant, such that each participant initially sees a same version of the virtual model.

2. The method of claim 1, further comprising:
obtaining an exploration request (152) for at least one of the participants; and
responsive to obtaining the exploration request, for each of the at least one participant associated with the exploration request, rendering (156) the virtual model in the respective participant location such that the participant sees a personalized version of the virtual model.

3. The method of claim 2, further comprising:
obtaining a manipulation instruction (154) associated with the at least one participant, the manipulation instruction describing a modification to the virtual model, and wherein the personalized version of the virtual model for the at least one participant is based on the manipulation instruction.

4. The method of claim 3, wherein the manipulation instruction comprises at least one of a scale change, an orientation change, and a viewpoint change.

5. The method of claim 2 or 3, wherein the personalized version of the virtual model for the at least one participant is further based on a modification permission parameter associated with the at least one participant, the modification permission parameter describing a limitation of the modification of the virtual model by the at least one participant.

6. The method of claim 5, wherein the modification permission parameter is based on a context of the meeting.

7. The method of claim 5 or 6, wherein the modification permission parameter is further based on a system map describing associations between different aspects of the virtual model.

8. The method of any of claims 2-6, wherein the mode instruction is obtained from a presenter, the presenter being a participant responsible for the meeting.

9. The method of claim 8, wherein the same version of the virtual model is based on a presenter manipulation instruction obtained from the presenter, the presenter manipulation instruction describing a modification to the virtual model.

10. The method of any of claims 1-9, wherein the mode instruction comprises at least one of the grouped visualization request, the personal visualization request and a mixed visualization request, the method further comprising:
responsive to obtaining the mode instruction comprising the mixed visualization request, for each of the participants, rendering (140) the virtual model in the respective participant location such that each participant initially sees a same version of the virtual model, and rendering the virtual model in the central location such that each participant also sees the same version of the virtual reality model.

11. The method of any of claims 1-10, further comprising:
responsive to obtaining the mode instruction comprising the personal visualization request, for at least one participant, rendering the virtual model in the participant locations corresponding to each of the participants, such that the at least one participant sees a view of the virtual model rendered for each of the participants in the respective participant locations.

12. The method of any of claims 1-11, wherein the virtual model represents an anatomical object, and optionally wherein the modification permission parameter is based on an anatomical system map describing associations between different aspects of the anatomical object.

13. A computer program comprising computer program code means adapted, when said computer program is run on a computer, to implement the method of any of claims 1-12.

14. A system (200) for generating virtual model renderings in extended reality for viewing by participants of a meeting, the system comprising:
an interface (210) configured to obtain a mode instruction comprising at least one of a grouped visualization request and a personal visualization request;
a central control element (220) configured, responsive to obtaining the mode instruction comprising a grouped visualization request via the interface, to render a virtual model in a central location corresponding to an area viewable by each of the participants, such that each participant sees a same version of the virtual model; and
a dispersed control element (230) configured, responsive to obtaining the mode instruction comprising the personal visualization request via the interface, for each of the participants, to render the virtual model in a respective participant location corresponding to an area in front of the participant, such that each participant initially sees a same version of the virtual reality model.

15. The system of claim 14, wherein the interface (210) is further configured to obtain an exploration request for at least one of the participants; and
a modification unit (240) configured, responsive to obtaining the exploration request via the interface, for each of the at least one participant associated with the exploration request, to render the virtual model in the respective participant location such that the participant sees a personalized version of the virtual model.
